# EUROPEAN PATENT SPECIFICATION

(11) **EP 3 494 965 B1**
(45) Date of publication and mention of the grant of the patent: **02.06.2021**
(21) Application number: 18185608.9
(22) Date of filing: 25.07.2018
(51) Int. Cl.: A61K 9/14, A61K 31/4418

(54) **AN IMPROVED PROCESS FOR THE PREPARATION OF PIRFENIDONE**
VERBESSERTES VERFAHREN ZUR HERSTELLUNG VON PIRFENIDON
PROCÉDÉ AMÉLIORÉ POUR LA PRÉPARATION DE PIRFÉNIDONE

(30) Priority: 11.12.2017 IN 201741044403
(43) Date of publication of application: 12.06.2019
(73) Proprietor: Laurus Labs Limited, 500078 Shareerpet, Hyderabad (IN)
(72) Inventor: MANDADAPU, Venkata Pramod Kumar, 500078 Sharmeerpet, Hyderabad, Telangana (IN); GALLA, Tirumala Rao, 500078 Sharmeerpet, Hyderabad, Telangana (IN); DADI, Jagadeeswara Rao, 500078 Sharmeerpet, Hyderabad, Telangana (IN); BOLLU, Ravindra Babu, 500078 Sharmeerpet, Hyderabad, Telangana (IN); VASIREDDI, Uma Maheswer Rao, 500078 Sharmeerpet, Hyderabad, Telangana (IN)
(74) Representative: Hamm&Wittkopp Patentanwälte PartmbB

(56) References cited:
- US-A1- 2012 192 861
- US-A1- 2017 281 609
- VARTIAINEN V. ET AL.: "Development of inhalable drug formulations for idiopathic pulmonary fibrosis", DRUG DELIVERY TO THE LUNGS, vol. 27, 2016, pages 1-4, XP002788088,

## Description

### FIELD OF THE INVENTION

The present invention generally relates to processes for particle size reduction of pirfenidone by wet milling techniques.

### BACKGROUND OF THE INVENTION

Pirfenidone is an anti-fibrotic drug for the treatment of idiopathic pulmonary fibrosis (IPF). It works by reducing lung fibrosis through down regulation of the production of growth factors and procollagens I and II. Pirfenidone was approved by USFDA as Oral/Capsule and Oral/Tablet for the treatment of idiopathic pulmonary fibrosis. Pirfenidone is chemically known as 5-methyl-1-phenyl-2-1(H)-pyridone and represented by the following structural formula (I):

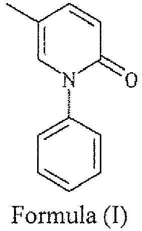

Idiopathic pulmonary fibrosis (IPF) is a rare disease of unknown etiology that is MVS:anm characterized by progressive fibrosis of the interstitium of the lung, leading to decreasing lung volume and progressive pulmonary insufficiency.

The process for the preparation of pirfenidone was first disclosed in US3839346 ("the '346 patent"). The disclosed process involves reaction of 5-methyl-2-(1H)-pyridone with iodo benzene in presence of anhydrous potassium carbonate and zinc precipitated copper powder to get pirfenidone. Further, this patent remains silent about particle size of obtained pirfenidone.

US patent No. 8519140 ("the '140 patent") disclosed an improved process for the preparation of pirfenidone and the obtained pirfenidone was milled through a loop mill in order to reduce the particle size to less than 150µm.

PCT. Application WO2017/122139 ("the '139 application") disclosed preparation of pirfenidone with particle size of D₉₀ 50-100µm by solvent and anti-solvent technique.

In pharmaceutical industry, particle characterization of powder materials has become one of the crucial aspects in drug product development and quality control of solid oral dosage forms. The particle size distribution (PSD) of the drug substance may have significant effects on final drug product performance (e.g., dissolution, bioavailability, content uniformity, stability, etc.). Furthermore, the PSDs of both drug substance and excipients can affect drug product manufacturability (e.g., flowability, blend uniformity, compactibility, etc.), which, ultimately, can impact safety, efficacy, and quality of the drug product. The PSDs of pharmaceutical powders have profound influence on almost every step of manufacturing processes for solid oral dosage forms, including pre-mixing/mixing, granulation, drying, milling, blending, coating, encapsulation, and compression. Therefore, the impact of particle sizes of pharmaceutical powders on drug product manufacturability and performance should be evaluated at different pharmaceutical development phases for each specific drug application.

The present inventors have found that by performing safety studies pirfenidone is a dust explosive, shock sensitive and having a very low minimum Ignition energy. Based on the studies, it was observed that pirfenidone is a St_{2H} class (violent explosion nature) with sparks and fumes and having very low minimum ignition energy of 1.7mJ, which indicates the material can ignite even at low ignition energy.

Particle size reduction process disclosed under the '140 Patent involves loop mill technique. A loop mill grinds materials by using a high speed jet of compressed air or inert gas in dry state to impact particles into each other. Dry milling process used in the loop mill particle size reduction always generates energetic particles that generate thermal energy which generally leads to explosion or burnings.

Based on these studies, inventors have realized that known dry mill technique is not appropriate selection for a material that has aforementioned drawbacks, for instance pirfenidone to avoid explosion due to static charge dissipation (Hammer Milling and Jet Milling Fundamentals Gary Liu, P.E. June, 2017).

Hence there is a need in the art to develop an alternative particle size reduction technique, which is suitable for sensitive materials that having a nature of dust explosive, shock sensitive and low ignition energy. Therefore the present invention provides alternative milling techniques in order to overcome the afore mentioned drawbacks. More specifically, the present invention provides wet mill techniques like colloid mill, ultra-sonication and high speed homogenizer techniques to reduce the particle size distribution of pirfenidone to below about 500 µm.

### SUMMARY OF THE INVENTION

The present invention provides wet milling techniques for particle size reduction, which are suitable for sensitive materials that are having properties like dust explosive, shock sensitive and low ignition energy. Specifically, the present invention provides a process for reduction of particle size of pirfenidone by wet mill techniques., as defined in the appended claims. More specifically the present invention provides a process for particle size reduction of pirfenidone to below about 500 µm by wet mill techniques, which process avoids the aforementioned drawbacks associated with the known dry milling techniques.

In accordance with one embodiment, the present invention provides a process for particle size reduction of pirfenidone by wet milling.

In accordance with another embodiment, the present invention provides a process for particle size reduction of pirfenidone by wet milling in water.

In accordance with another embodiment, the present invention provides a process for particle size reduction of pirfenidone by wet milling using a suitable apparatus selected from colloid mill, ultrasonicator or high speed homogenizer.

In accordance with another embodiment, the present invention provides a process for particle size reduction of pirfenidone by wet milling using a suitable apparatus selected from colloid mill, ultrasonicator or high speed homogenizer in water.

In accordance with another embodiment, the present invention provides a process for particle size reduction of pirfenidone having a particle size distribution of above about 500 µm, by wet milling.

In accordance with another embodiment, the present invention provides a process for particle size reduction of pirfenidone having a particle size distribution of above about 500 µm, by wet milling using a suitable apparatus selected from colloid mill, ultrasonicator or high speed homogenizer.

In accordance with another embodiment, the present invention provides a process for particle size reduction of pirfenidone having a particle size distribution of above about 500 µm, by wet milling using a suitable apparatus selected from colloid mill, ultrasonicator or high speed homogenizer in water.

In accordance with another embodiment, the present invention provides a process for particle size reduction of pirfenidone having a particle size distribution of above about 500 µm, by wet milling; comprising: mixing pirfenidone having a particle size distribution of above about 500 µm in water for a sufficient period of time, and recovering the pirfenidone having a particle size distribution of below 500 µm.

In accordance with another embodiment, the present invention provides a process for particle size reduction of pirfenidone having a particle size distribution of above about 500 µm, by wet milling using a suitable apparatus; comprising: mixing pirfenidone having a particle size distribution of above about 500 µm in water for a sufficient period of time, and recovering the pirfenidone having a particle size distribution of below 500 µm; wherein the suitable apparatus is selected from colloid mill, ultrasonicator or high speed homogenizer.

In accordance with another embodiment, the present invention provides a process for particle size reduction of pirfenidone by wet milling using colloid mill.

In accordance with another embodiment, the present invention provides a process for particle size reduction of pirfenidone by wet milling using colloid mill in water.

In accordance with another embodiment, the present invention provides a process for particle size reduction of pirfenidone having a particle size distribution of above about 500 µm, by wet milling using colloid mill.

In accordance with another embodiment, the present invention provides a process for particle size reduction of pirfenidone having a particle size distribution of above about 500 µm, by wet milling using colloid mill in water.

In accordance with another embodiment, the present invention provides a process for particle size reduction of pirfenidone having a particle size distribution of above about 500 µm, by wet milling; comprising:
a) mixing pirfenidone having a particle size distribution of above about 500 µm with water,
b) circulating the step a) mixture in to colloidal mill, and
c) recovering the pirfenidone.

In accordance with another embodiment, the present invention provides a process for particle size reduction of pirfenidone having a particle size distribution of above about 500 µm, by wet milling using ultrasonicator.

In accordance with another embodiment, the present invention provides a process for particle size reduction of pirfenidone having a particle size distribution of above about 500 µm, by wet milling using ultrasonicator in water.

In accordance with another embodiment, the present invention provides a process for particle size reduction of pirfenidone having a particle size distribution of above about 500 µm, by wet milling; comprising:
a) mixing pirfenidone having a particle size distribution of above about 500 µm with water,
b) sonicating the step a) mixture in a ultrasonicator, and
c) recovering the pirfenidone.

In accordance with another embodiment, the present invention provides a process for particle size reduction of pirfenidone having a particle size distribution of above about 500 µm, by wet milling using high speed homogenizer.

In accordance with another embodiment, the present invention provides a process for particle size reduction of pirfenidone having a particle size distribution of above about 500 µm, by wet milling using high speed homogenizer in water.

In accordance with another embodiment, the present invention provides a process for particle size reduction of pirfenidone having a particle size distribution of above about 500 µm, by wet milling; comprising:
a) mixing pirfenidone having a particle size distribution of above about 500 µm with water in a high speed homogenizer,
b) stirring the step a) mixture, and
c) recovering the pirfenidone.

In accordance with another embodiment, the present invention provides a process for particle size reduction of pirfenidone having a particle size distribution of above about 500 µm, by wet milling using a suitable apparatus; comprising: mixing pirfenidone having a particle size distribution of above about 500 µm in water for a sufficient period of time, and recovering the pirfenidone having a particle size distribution of above about 10 µm and below about 500 µm; wherein the suitable apparatus is selected from colloid mill, ultrasonicator or high speed homogenizer.

In accordance with another embodiment, the present invention provides a process for particle size reduction of pirfenidone having a particle size distribution of above about 500 µm, by wet milling; comprising:
a) mixing pirfenidone having a particle size distribution of above 500 µm with water,
b) circulating the step a) mixture in to colloidal mill, and
c) recovering the pirfenidone having a particle size distribution of above about 10 µm and below about 500 µm.

Further, a process for preparing pirfenidone having a particle size D90 of above 500 microns, preferably of 1000-3500 µm, more preferred 2000-3000 microns, comprising:
a) dissolving pirfenidone in an organic solvent at higher temperatures, preferably at a temperature of 40°C to the boiling point of the organic solvent, and
b) cooling the solution obtained in step (a) to below 30°C,
is claimed.

In accordance with another embodiment, the present invention provides a pharmaceutical composition comprising pirfenidone prepared by the process of the invention and at least one pharmaceutically acceptable excipient.

### DETAILED DESCRIPTION OF THE INVENTION

The present invention provides wet milling techniques for particle size reduction, which are suitable for sensitive materials that are having properties like dust explosive, shock sensitive and low ignition energy. Specifically, the present invention provides a process for particle size reduction of pirfenidone by wet mill techniques. More specifically the present invention provides a process for particle size reduction of pirfenidone to below about 500 µm by wet mill techniques, which process avoids the aforementioned drawbacks associated with the known dry milling techniques.

The known milling processes involve dry milling of pirfenidone by loop mill method. The loop mill grinds materials by using a high speed jet of compressed air or inert gas in dry state to impact particles into each other. Dry milling process used in the loop mill particle size reduction always generates an energetic particle that generates thermal energy which generally leads to explosion or burnings. The present milling process involves milling of pirfenidone in wet medium preferably in aqueous medium, wherein the thermal energy generated by the energetic particles is absorbed by the moisture on the particle surfaces and thereby preventing explosion or burning caused by localized hot spots. Thus, the process is safer than the dry milling processes, particularly in the commercial scale operations.

As used herein, the term "particle size distribution" is expressed in terms of D₉₀, D₅₀ and D₁₀, which corresponds to the diameter of 90 percent by volume of the particles, 50 percent by volume of the particles and 10 percent by volume of the particles are present, respectively.

As used herein, the term "recovering" refers to a process of obtaining pirfenidone by means of filtration, decantation, extraction, distillation, evaporation, centrifugation or a combination thereof.

As used herein, the term "mixing" refers to suspending pirfenidone in a medium.

As used herein, the term "about" refers to a variation of 10% from the indicated values, or in case of a range of values, means a 10% variation from both the lower and upper limits of such ranges.

As used herein, the starting material pirfenidone having a particle size distribution of above about 500 µm may be obtained from any processes known in the art; preferably the starting material used herein is obtained from reference examples 4 & 5.

In accordance with one embodiment, the present invention provides a process for particle size reduction of pirfenidone by wet milling.

In another embodiment, the present invention provides a process for particle size reduction of pirfenidone by wet milling using a suitable apparatus selected from colloid mill, ultrasonicator or high speed homogenizer.

In another embodiment, the present invention provides a process for particle size reduction of pirfenidone by wet milling in water using a suitable apparatus selected from colloid mill, ultrasonicator or high speed homogenizer.

In another embodiment, the present invention provides a process for particle size reduction of pirfenidone having a particle size distribution of above about 500 µm, by wet milling using a suitable apparatus selected from colloid mill, ultrasonicator or high speed homogenizer.

### Colloid mill:

In a specific embodiment, the present invention provides a process for particle size reduction of pirfenidone by wet milling using colloid mill.

In accordance with another embodiment, the present invention provides a process for particle size reduction of pirfenidone by wet milling using colloid mill in water.

In accordance with another embodiment, the present invention provides a process for particle size reduction of pirfenidone having a particle size distribution of above about 500 µm, by wet milling using colloid mill.

In accordance with another embodiment, the present invention provides a process for particle size reduction of pirfenidone having a particle size distribution of above about 500 µm, by wet milling; comprising:
a) mixing pirfenidone having a particle size distribution of above about 500 µm with water,
b) circulating the step a) mixture in to colloidal mill, and
c) recovering the pirfenidone.

Colloid mill is a machine used in the process of milling of solid particles to reduce size of the particles or making homonization of the solid particles present in suspension or emulsion. Colloid mill instrument and method of milling for the use of pharmaceutical products is known from the art; for example Colloid mill instrument used in the present invention is having following identification: Colloid mill/IKA MK 2000/05 or any equivalent colloid mill instrument can be used as long as pirfenidone with required particle size is produced during the process of the invention.

The input pirfenidone may be any crystalline or other form of pirfenidone with any particle size preferably above about 500 µm, including various solvates, hydrates, salts and co-crystals as long as pirfenidone with required particle size is produced during the process of the invention or pirfenidone obtaining as existing slurry from a previous processing step.

The aforementioned step a) process involves mixing pirfenidone having a particle size distribution of above about 500 µm with water. Then the resultant step a) mixture is circulated in to colloid mill. The circulation temperature should be sufficient to form the required particle size. Typically the circulation temperature can be from about 5°C to about 65°C; preferably at about 25°C to about 50°C.

Reduction of particle size of the invention is dependent on the number of revolutions per minute (rpm) of the colloid mill and duration of circulation of the reaction suspension. The rpm and the circulation time are not particularly limited to specific values but an exemplary the same may be employed in the range of about 500 to about 10000 rpm for a period of about 5 minutes to about 30 hours; preferably about 1000 to about 8000 rpm for a period of about 30 minutes to about 24 hours.

After attaining the required particle size, preferably to below 500 µm, the resultant product may be recovered by known methods, for example decantation, filtration and the like; preferably by filtration. Optionally before recovery of the product, the reaction mixture may be allowed to cool to about 0°C to about 15°C and stirred for about 30 min to 10 hours at same temperature. The resultant product may be dried using conventional methods known in the art at a temperature ranging from about 40°C to about 75°C for a period of 2 hours to 10 hours. Preferably drying is carried out at about 50°C to about 65°C for a period of 4 hours to 6 hours.

Pirfenidone particles obtained by the reduction process using colloid mill is having a particle distribution of about D₁₀ less than 50 µm, preferably less 20 µm; D₅₀ less than 100 µm, preferably 50 µm; D90 less than 200 µm, preferably 100 µm.

### Ultrasonication:

In another embodiment, the present invention provides a process for particle size reduction of pirfenidone having a particle size distribution of above about 500 µm, by wet milling using ultrasonicator.

In another embodiment, the present invention provides a process for particle size reduction of pirfenidone having a particle size distribution of above about 500 µm, by wet milling using ultrasonicator in water.

In a preferred embodiment, the present invention provides a process for particle size reduction of pirfenidone having a particle size distribution of above about 500 µm, by wet milling; comprising:
a) mixing pirfenidone having a particle size distribution of above about 500 µm with water,
b) sonicating the step a) mixture in a ultrasonicator, and
c) recovering the pirfenidone.

Ultrasonication is an efficient means for the wet-milling and micro-grinding of particles. In particular for the manufacturing of superfine-size slurries, ultrasonication has many advantages, when compared with common size reduction equipment, such as: ball mills, bead mills, disc mills or jet mills. Ultrasonication allows for the processing of high-concentration and high-viscosity slurries - therefore reducing the volume to be processed. Ultra sonic waves do not allow agglomerates to form as observed during conventional crystallization process. Accordingly, sonication is a safe process that allows easy scale up based on constant power per unit volume.

The input pirfenidone may be any crystalline or other form of pirfenidone with any particle size preferably above about 500 µm, including various solvates, hydrates, salts and cocrystals as long as pirfenidone with required particle size is produced during the process of the invention or pirfenidone obtaining as existing slurry from a previous processing step.

The step of mixing pirfenidone having a particle size distribution of above about 500 µm with water is placed in an ultrasonicator and sonicating for a sufficient period of time. The ultrasonicator temperature should be sufficient to form the required particle size. Typically the sonication temperature can be from about 5°C to about 65°C for a period of about 30 min to about 10 hours. Preferably the sonication temperature is about 25°C to about 50°C for a period of about 1 hour to about 5 hours.

After attaining the required particle size, preferably to below 500 µm, the resultant product may be recovered by known methods, for example decantation, filtration and the like; preferably by filtration. Optionally before recovery of the product, the reaction mixture may be allowed to cool to about 0°C to about 15°C and stirred for about 30 min to 10 hours at same temperature. The resultant product may be dried using conventional methods known in the art at a temperature ranging from about 40°C to about 75°C for a period of 2 hours to 10 hours. Preferably drying is carried out at about 50°C to about 65°C for a period of 4 hours to 6 hours.

Pirfenidone particles obtained by the reduction process after ultrasonication is having a particle distribution of about D₁₀ less than 50 µm, preferably less 30 µm; D₅₀ less than 150 µm, preferably 100 µm; D90 less than 300µm, preferably 250 µm.

### High speed homogenizer:

In another embodiment, the present invention provides a process for particle size reduction of pirfenidone having a particle size distribution of above about 500 µm, by wet milling using high speed homogenizer.

In another embodiment, the present invention provides a process for particle size reduction of pirfenidone having a particle size distribution of above about 500 µm, by wet milling using high speed homogenizer in water.

In a preferred embodiment, the present invention provides a process for particle size reduction of pirfenidone having a particle size distribution of above about 500 µm, by wet milling; comprising:
a) mixing pirfenidone having a particle size distribution of above about 500 µm with water in a high speed homogenizer,
b) stirring the step a) mixture, and
c) recovering the pirfenidone.

The input pirfenidone may be any crystalline or other form of pirfenidone with any particle size preferably above about 500 µm, including various solvates, hydrates, salts and cocrystals as long as pirfenidone with required particle size is produced during the process of the invention or pirfenidone obtaining as existing slurry from a previous processing step.

The step of mixing pirfenidone having a particle size distribution of above about 500 µm with water is placed in high speed homogenizer and stir for a sufficient period of time. The high speed homogenizer temperature should be sufficient to form the required particle size. Typically the stirring temperature can be from about 5°C to about 65 °C for a period of about 30 min to about 10 hours. Preferably the stirring temperature is about 25°C to about 50°C for a period of about 1 hour to about 5 hours.

The stirring speed of the reaction mixture is not particularly limited but may be employed in the range of 500-10000 rounds per minute, preferably 1000-7000 rounds per minute.

After attaining the required particle size, preferably to below 500 µm, the resultant product may be recovered by known methods, for example decantation, filtration and the like; preferably by filtration. Optionally before recovery of the product, the reaction mixture may be allowed to cool to about 0°C to about 15°C and stirred for about 30 min to 10 hours at same temperature. The resultant product may be dried using conventional methods known in the art at a temperature ranging from about 40°C to about 75°C for a period of 2 hours to 10 hours. Preferably drying is carried out at about 50°C to about 65°C for a period of 4 hours to 6 hours.

The resultant product may be further dried using conventional methods known in the art at a temperature ranging from about 40°C to about 75°C for a period of 2 hours to 10 hours. Preferably drying is carried out at about 50°C to about 65°C for a period of 4 hours to 6 hours.

Pirfenidone particles obtained by the reduction process after high speed homogenizer is having a particle distribution of about D₁₀ less than 50 µm, preferably less 30 µm; D₅₀ less than 150 µm, preferably 100 µm; D90 less than 300µm, preferably 250 µm.

In another embodiment, the present invention provides a pharmaceutical composition, comprising pirfenidone prepared by the processes of the present invention and at least one pharmaceutically acceptable excipient. Such pharmaceutical composition may be administered to a mammalian patient in any dosage form, e.g., solid, liquid, powder, injectable solution, etc.

The particle size distribution of pirfenidone is measured utilizing: Instrument model: Malvern; Mastersizer 3000; particle refraction index of sample: 1.700; Absorption: 0.01 and Analysis model: general purpose, normal sensitivity, non-spherical particles.

Pirfenidone having a particle size D90 of above 500 microns, which may be used in the particle size reduction process of the present invention, may be obtained by dissolving pirfenidone in an organic solvent at higher temperatures, preferably at a temperature of 40°C to the boiling point of the organic solvent, and cooling the obtained solution to below 30°C. The crystallized product is finally separated off by filtration.

Examples of the organic solvent include carboxylic acid esters, such as isopropyl acetate, methyl acetate and ethyl acetate, alcohols, such as methanol, ethanol, isopropanol and n-propanol, ketones, such as acetone, methyl ethyl ketone, methyl isobutyl ketone, ethers, such as methyl tert-butyl ether, tetrahydrofuran, dimethyl ether, diisopropyl ether and 1,4-dioxane, and mixtures thereof. Preferably, the organic solvent is a C₁₋₄-alkyl acetate, most preferably ethyl acetate.

### EXAMPLES

The following non limiting examples illustrate specific embodiments of the present invention.

### EXAMPLE-1:

Particle size reduction of pirfenidone using colloid mill (IKA MK 2000/05)

Pirfenidone (Particle size distribution: D₁₀: 1330 µm; D₅₀: 1950 µm and D₉₀: 2840 µm) and Water (3 vol) were charged into IKA colloid mill at 25°C to 35°C and circulated the reaction mass at 25°C to 35°C till required particle size was obtained. Particle size distribution of the sample with different time interval was monitored by collecting the sample from reaction mass. After achieving the required particle size, stirred the reaction mixture for 30-60 min at 25°C to 35°C. Further, the reaction mixture temperature was allowed to cool to 0°C to 5°C and stirred for 2-3 hrs at same temperature. Filter the solid and washed with the wet cake with chilled water (0.5 vol), dried the solid in an oven under vacuum at 60°C for 4-6 hours.

The following examples were performed using the above described process with different circulation time and different rpm and obtained results are tabulated as below:

**Table 1:**

| **Ex. No.** | **Input** | **Out put** | **rpm** | **Circulation time** | **PSD D₁₀** | **PSD D₅₀** | **PSD D₉₀** |
|---|---|---|---|---|---|---|---|
| 1A | 20 kg | 17 kg | 6000 | 1 hour | 30 µm | 105 µm | 206 µm |
| | | | | 2 hours | 28 µm | 72 µm | 165 µm |
| | | | | 3 hours | 26 µm | 61 µm | 143 µm |
| | | | | 4 hours | 23 µm | 54 µm | 129 µm |
| | | | | 5 hours | 22 µm | 53 µm | 117 µm |
| | | | | 6 hours | 21 µm | 49 µm | 107 µm |
| 1B | 200 kg | 180kg | 6000 | 45 min | 41 µm | 48 µm | 285 µm |
| | | | | 2 hours | 34 µm | 124 µm | 257 µm |
| | | | | 3 hours | 34 µm | 116 µm | 236 µm |
| | | | | 4 hours | 32 µm | 106 µm | 222 µm |
| | | | | 5 hours | 33 µm | 106 µm | 217 µm |
| | | | | 6 hours | 29 µm | 101 µm | 200 µm |
| | | | | 7 hours | 31 µm | 94 µm | 200 µm |
| | | | | 8 hours | 28 µm | 84 µm | 187 µm |
| | | | | 9 hours | 28 µm | 80 µm | 180 µm |
| | | | | 10 hours | 27 µm | 69 µm | 155 µm |
| | | | | 12 hours | 25 µm | 61 µm | 150 µm |
| 1C | 80 kg | 71kg | 6000 | 8 hours | 21 µm | 57 µm | 132 µm |
| | | | | 10 hours | 22 µm | 55 µm | 122 µm |
| | | | | 12 hours | 22 µm | 54 µm | 121 µm |
| | | | | 14 hours | 21 µm | 47 µm | 98 µm |
| | | | | 16 hours | 21 µm | 47 µm | 96 µm |
| | | | | 19 hours | 22 µm | 46 µm | 93 µm |
| 1D | 200 gr | 180 gr | 3000 | 30 min | 28 µm | 87 µm | 185 µm |
| 1E | 200 gr | 175 gr | 3000 | 60 mim | 12 µm | 33 µm | 123 µm |
| 1F | 200 gr | 170 gr | 3000 | 120 mim | 13 µm | 33 µm | 82 µm |
| 1G | 200 gr | 185 gr | 5000 | 5 min | 11 µm | 40 µm | 99 µm |
| 1H | 200 gr | 175 gr | 5000 | 60 min | 11 µm | 36 µm | 89 µm |
| 1I | 200 gr | 175 gr | 8000 | 5 min | 9 µm | 35 µm | 85 µm |

### EXAMPLE-2:

Particle size reduction of pirfenidone using high speed homogenizer (Silverson L5MA Lab Mixer)

Water (300 mL) and Pirfenidone (100gr; Particle size distribution: D₁₀: 1330 µm; D₅₀: 1950 µm and D₉₀: 2840 µm) were charged into reaction vessel at 25°C to 35°C and was placed High speed stirrer/Homogenizer into the reaction mass till required particle size was obtained at 25°C to 35°C. Particle size distribution of the sample with different time interval was monitored by collecting the sample from reaction mass. After achieving the required particle size, stirred the reaction mixture for 30-60 min at 25 °C to 35°C. Further, the reaction mixture temperature was allowed to cool to 0°C to 5°C and stirred for 2-3 hrs at same temperature. Filter the solid and washed with the wet cake with chilled water (100 mL), dried the solid in an oven under vacuum at 60°C for 4-6 hours.

The following examples were performed using the above described process with different rpm and time; the obtained results are tabulated below:

**Table 2:**

| **Exp. No** | **Input Wt.** | **Output Wt.** | **RPM** | **Stirring time** | **PSD D₁₀** | **PSD Dso** | **PSD D₉₀** |
|---|---|---|---|---|---|---|---|
| 2A | 100 g | 90 g | 2000 | 30 min | 24 µm | 82 µm | 230 µm |
| 2B | 100 g | 87 g | | 60 min | 24 µm | 88 µm | 200 µm |
| 2C | 100 g | 84 g | | 120 min | 21 µm | 75 µm | 165 µm |
| 2D | 100 g | 80 g | | 240 min | 21 µm | 71 µm | 154 µm |
| 2E | 100 g | 85 g | 5000 | 60 min | 17 µm | 48 µm | 108 µm |
| 2F | 100 g | 86 g | 7000 | 60 min | 13 µm | 42 µm | 91 µm |

### EXAMPLE-3:

Particle size reduction of pirfenidone using ultra sonication by sonoprocessor (RTUL 10SP-20-3000-SD)
Water (300 mL) and Pirfenidone (200gr; Particle size distribution: D₁₀: 1330 µm; D₅₀: 1950 µm and D₉₀: 2840 µm) were charged into reaction vessel at 25°C to 35°C and was placed Sonoprocessor into the reaction mass till required particle size was obtained at 25°C to 35°C. Particle size distribution of the sample with different time interval was monitored by collecting the sample from reaction mass. After achieving the required particle size, stirred the reaction mixture for 30-60 min at 25°C to 35°C. Further, the reaction mixture temperature was allowed to cool to 0°C to 5°C and stirred for 2-3 hrs at same temperature. Filter the solid and washed the wet cake with chilled water (100 mL), dried the solid in an oven under vacuum at 60°C for 4-6 hours.

The following examples were performed using the above described process with different rpm and time; the obtained results are tabulated below:

**Table 3:**

| **Exp. No** | **Input Wt.** | **Output Wt.** | **Sonication time** | **PSD D₁₀** | **PSD D₅₀** | **PSD D₉₀** |
|---|---|---|---|---|---|---|
| 3A | 200 g | 183 g | 30 min | 44 µm | 145 µm | 245 µm |
| 3B | 200 g | 180 g | 60 min | 26 µm | 101 µm | 189 µm |
| 3C | 200 g | 181 g | 120 min | 19 µm | 68 µm | 150 µm |
| 3D | 200 g | 175 g | 180 min | 13 µm | 48 µm | 109 µm |

### Reference Example 1:

### Dust explosion test by Modified Hartmann Apparatus:

This test was performed based on procedures from VDI 2263, Part 1 (Section 2.1.1), "Dust fires and dust explosions; hazards, assessment, protective measures", using a Modified Hartmann Apparatus. Pirfenidone was dried at 50°C under vacuum, sieved through 63µ sieve. Weighed amount of product was charged into a modified hartmann tube of 1.2 liter volume. The product was converted into a dust cloud in glass cylindrical tube with compressed air and electrodes with continuous spark was used as ignition source to ignite the dust cloud. Based on dust fire or extent of opening of the hinged cover rating 0, 1 or 2 are assigned.

| **Observation** | **Class** |
|---|---|
| No fire or explosion | St _{0H} |
| Dust fire or mild explosion | St _{1H} |
| Violent explosion | St _{2H} |

**The obtained results are tabulated:**

| **S. No** | **Wt. of sample (mg)** | **Eq. dust concentration (g/m3)** | **Class** |
|---|---|---|---|
| 1 | 120 | 100 | 1 |
| 2 | 240 | 200 | 2 |

### Reference Example 2:

### Shock sensitive test by Fall hammer test:

This test was performed by Lutoif/ESCIS method using swissi process safety GmbH falling hammer MP03. The hammer (weight 5 kg) was raised and fixed at a height of 80 cm. A Pirfenidone capsule was placed on the lower part of the stamp. The upper stamp was placed on the sample. The hammer was released by using release lever. Result: Detonation was observed (spart and/or smoke/fumes).

### Reference Example 3:

### Minimum Ignition Energy test by MIKE3:

This test was performed based on international test method VDI 2263 part 1.2.5 using MIKE3. Pirfenidone was dried at 50°C under vacuum, sieved through 63µ sieve. The product was converted into a dust cloud in glass cylindrical tube with compressed air. The spark for ignition was provided with moving electrodes assembly at different energy levels from 1 mJ to 1000 mJ. The energy just sufficient to ignite the dust under ignition was determined. This ignition energy was then successively halved with variation of the dust concentration and the ignition delay time in a series of tests until no ignition takes place in at least 10 successive experiments. The minimum ignition energy MIE lies between the lowest energy value at which ignition occurred and at the energy value at which no ignition observed for 10 successive experiments. The recommendations for interpreting MIE results are based on energy levels available on MIKE 3 apparatus. According to usual practice, MIE can be ranked as follow:

| **Observation** | **Result** |
|---|---|
| MIE> 1000mJ | Sample almost insensitive to electrostatic ignition |
| 300 mJ < MIE < 1000 mJ, 100 mJ < MIE < 300 mJ and 30 mJ < MIE < 100 mJ | Sample sensitive to electrostatic ignition |
| 10 mJ < MIE < 30 mJ and 3 mJ < MIE < 10 mJ | Sample very sensitive to electrostatic ignition |
| 1 mJ < MIE < 3 mJ and MIE < 1 mJ | sample extremely sensitive to electrostatic ignition |

**Results:** 1 mJ< MIE < 3 mJ/ Es = 1.7 mJ.

### Reference Example 4:

### Preparation of pirfenidone:

A mixture of 5-methyl-1H-pyridin-2-one (100 g), bromo benzene (259 g, comprising greater than 0.2% by weight dibromobenzene isomers) and dimethylformamide (200 ml) were added in to a round bottom flask and stirred up to complete dissolution. Potassium carbonate (254 g) and copper (I) chloride (18.2 g) was added to the above reaction mass and then heated to 130-140°C. The reaction mass was stirred at 130-140°C for 10 hrs. After the reaction completion, the reaction mass was cooled to 25-35°C. Toluene (500 ml), aqueous sodium chloride (75 g of sodium chloride in 500 ml of water) was added to the reaction mass and stirred for 15-30 mins at 25-35°C. The reaction mass was filtered and the filtrate was allowed to settle. Organic and aqueous layers were separated and the aqueous layer was extracted with toluene. Organic layers combined and was washed with aqueous sodium chloride, treated with carbon and filtered through hyflo. The solvent from the filtrate was distilled off completely under vacuum at below 60°C. Toluene (300 ml) was added to the obtained residue and stirred for 30 mins. The reaction mass was heated to 77-83°C and stirred for 45 mins. The reaction mass was cooled to 25-35°C over 60 mins. The reaction mass was further cooled to 0-6°C. The solid obtained was filtered, washed with toluene and dried under vacuum. DM water (500 ml) was added to the above obtained wet compound followed by 50% aqueous sodium hydroxide solution (10 g of sodium hydroxide in 20 ml of water) at 25-35°C. The reaction mass was heated to 75-85°C and stirred for 30-60 mins. The reaction mass was then gradually cooled to 25-35°C and stirred for 60 mins. The reaction mass was further cooled to 0-5°C and stirred for 3 hrs. The obtained solid was filtered, washed with water and dried to provide the title compound.

### Reference Example 5:

### Purification of Pirfenidone

Dissolving crude Pirfenidone (50g) in ethyl acetate (100 mL) at 65-75°C and the reaction mass was treated with activated carbon (5g) and filtered through a short bed of Hyflo. The filtrate was partially concentrated, under reduced pressure while maintain temperature below 60°C. The mixture was gradually allowed to cool to 30±5°C and then stirred for another 30-60 min. The suspension was again allowed to cool to 0-5°C and maintained for another 2-3h, at the same temperature. The precipitated material was filtered and then dried at 60±5°C, for 6-8h, to afford Pirfenidone as white colored powder (HPLC purity 99.9%; dimer content 0.03%). Yield: 45 g.
Particle size distribution: D10: 1330 µm, D50: 1950 µm, D90: 2840 µm

## Claims

1. A process for particle size reduction of pirfenidone having a particle size D90 of above 500 µm prior to particle size reduction by wet milling.

2. The process as claimed in claim 1, wherein the wet milling is carried out by using a suitable apparatus selected from colloid mill, ultrasonicator or high speed homogenizer.

3. The process as claimed in claim 1, wherein the wet milling is carried out in water.

4. The process as claimed in claim 1 comprising: mixing pirfenidone in water in a suitable apparatus selected from colloid mill, ultrasonicator or high speed homogenizer for a sufficient period of time, and recovering the pirfenidone.

5. The process as claimed in claim 4, wherein the pirfenidone after wet milling has a particle size D90 of below 500 µm.

6. The process as claimed in claim 1; comprising:
a) mixing pirfenidone with water,
b) circulating the step a) mixture in to colloidal mill, and
c) recovering the pirfenidone.

7. The process as claimed in claim 6, wherein the circulation comprises with 1000 to 8000 revolutions per minute (rpm).

8. The process as claimed in claim 6, wherein the circulation time is about 30 minutes to about 24 hours.

9. The process as claimed in claim 1 comprising:
a) mixing pirfenidone with water,
b) sonicating the step a) mixture in a ultrasonicator, and
c) recovering the pirfenidone.

10. The process as claimed in claim 9, wherein the sonication is carried out at a temperature of about 25°C to about 50°C for a period of about 1 hour to about 5 hours.

11. The process as claimed in claim 1 comprising:
a) mixing pirfenidone with water in a high speed homogenizer,
b) stirring the step a) mixture, and
c) recovering the pirfenidone.

12. The process as claimed in claim 11, wherein the stirring is carried out at a temperature of about 25°C to about 50°C for a period of about 1 hour to about 5 hours.

13. The process as claimed in claims 6-12, wherein the pirfenidone after wet milling has a particle size D90 of below 500 µm.

14. Pirfenidone having a particle size distribution of D10 less than about 50 µm, D50 less than about 150 µm and D90 less than about 300 µm, obtained by the processes according to claims 1- 13.

## Patentansprüche

1. Verfahren zur Partikelgrößenreduktion von Pirfenidon mit einer Partikelgröße von D90 über 500 µm vor der Partikelgrößenreduktion durch Nassmahlen.

2. Verfahren, wie in Anspruch 1 beansprucht, wobei das Nassmahlen in einer geeigneten Apparatur durchgeführt wird, ausgewählt aus einer Kolloidmühle, einem Ultraschallgerät oder einem Hochgeschwindigkeits-Homogenisator.

3. Verfahren, wie in Anspruch 1 beansprucht, wobei das Nassmahlen mit Wasser durchgeführt wird.

4. Verfahren, wie in Anspruch 1 beansprucht, umfassend: Mischen von Pirfenidon mit Wasser in einem geeigneten Apparat, ausgewählt aus einer Kolloidmühle, einem Ultraschallgerät oder einem Hochgeschwindigkeits-Homogenisator für eine ausreichende Zeitspanne und Rückgewinnung des Pirfenidons.

5. Verfahren, wie in Anspruch 4 beansprucht, wobei Pirfenidon nach dem Nassmahlen eine Partikelgröße D90 von weniger als 500 µm hat.

6. Verfahren, wie in Anspruch 1 beansprucht, umfasst
a) Mischen von Pirfenidon mit Wasser,
b) Zirkulieren des Gemisches aus Schritt a) in einer Kolloidmühle und
c) Rückgewinnung des Pirfenidons.

7. Verfahren, wie in Anspruch 6 beansprucht, wobei die Zirkulation 1000 bis 8000 Umdrehungen pro Minute (rpm) umfasst.

8. Verfahren, wie in Anspruch 6 beansprucht, wobei die Zirkulation ca. 30 Minuten bis ca. 24 Stunden dauert.

9. Verfahren, wie in Anspruch 1 beansprucht, umfassend:
a) Mischen von Pirfenidon mit Wasser,
b) Ultraschallbehandlung des Gemisches aus Schritt a) in einem Ultraschallgerät und
c) Rückgewinnung von Pirfenidon.

10. Verfahren, wie in Anspruch 9 beansprucht, wobei die Ultraschallbehandlung bei einer Temperatur von ca. 25°C bis ca. 50°C für eine Zeitspanne von ca. 1 h bis ca. 5 h durchgeführt wird.

11. Verfahren, wie in Anspruch 1 beansprucht, umfassend:
a) Mischen von Pirfenidon mit Wasser in einem Hochgeschwindigkeits-Homogenisator,
b) Rühren des Gemisches aus Schritt a) und
c) Rückgewinnung von Pirfenidon.

12. Verfahren, wie in Anspruch 11 beansprucht, wobei das Rühren bei einer Temperatur von ca. 25°C bis ca. 50°C für eine Zeitspanne von ca. 1h bis ca. 5 h durchgeführt wird.

13. Verfahren, wie in den Ansprüchen 6-12 beansprucht, wobei Pirfenidon nach dem Nassmahlen eine Partikelgröße D90 von weniger als 500 µm aufweist.

14. Pirfenidon mit einer Partikelgrößenverteilung von D10 weniger als ca. 50 µm, D50 weniger als ca. 150 µm und D90 weniger als ca. 300 µm, erhältlich durch ein Verfahren gemäß den Ansprüchen 1-13.

## Revendications

1. Procédé de réduction de granulométrie de pirfénidone ayant une granulométrie D90 supérieure à 500 µm avant la réduction de la granulométrie par broyage par voie humide.

2. Procédé selon la revendication 1, dans lequel le broyage par voie humide est effectué en utilisant un appareil approprié choisi parmi un broyeur colloïdal, un dispositif d'ultrasonication ou un homogénéisateur à grande vitesse.

3. Procédé selon la revendication 1, **caractérisé en ce que** le broyage par voie humide est réalisé dans de l'eau.

4. Procédé selon la revendication 1, comprenant: un mélange de pirfénidone dans de l'eau dans un appareil approprié choisi parmi un broyeur colloïdal, un dispositif d'ultrasonication ou un homogénéisateur à grande vitesse pendant une période de temps suffisante, et une récupération de la pirfénidone.

5. Procédé selon la revendication 4, **caractérisé en ce que** la pirfénidone, après broyage par voie humide, a une granulométrie D90 inférieure à 500 µm.

6. Procédé selon la revendication 1 ; comprenant :
a) un mélange de la pirfénidone avec de l'eau,
b) une circulation du mélange de l'étape a) dans le broyeur colloïdal, et
c) une récupération de la pirfénidone.

7. Procédé selon la revendication 6, **caractérisé en ce que** la circulation comprend 1 000 à 8 000 tours par minute (tpm).

8. Procédé selon la revendication 6, dans lequel le temps de circulation est d'environ 30 minutes à environ 24 heures.

9. Procédé selon la revendication 1 comprenant :
a) un mélange de la pirfénidone avec de l'eau,
b) une sonication du mélange de l'étape a) dans un dispositif d'ultrasonication, et
c) une récupération de la pirfénidone.

10. Procédé selon la revendication 9, dans lequel la sonication est effectuée à une température comprise entre environ 25°C et environ 50°C pendant une période d'environ 1 heure à environ 5 heures.

11. Procédé selon la revendication 1 comprenant :
a) un mélange de la pirfénidone avec de l'eau dans un homogénéisateur à grande vitesse,
b) une agitation du mélange de l'étape a), et
c) une récupération de la pirfénidone.

12. Procédé selon la revendication 11, dans lequel l'agitation est effectuée à une température comprise entre environ 25°C et environ 50°C pendant une durée d'environ 1 heure à environ 5 heures.

13. Procédé selon les revendications 6 à 12, dans lequel la pirfénidone, après broyage par voie humide, a une granulométrie D90 inférieure à 500 µm.

14. Pirfénidone ayant une distribution granulométrique D10 inférieure à environ 50 µm, D50 inférieure à environ 150 µm, et D90 inférieure à environ 300 µm, obtenue par les procédés selon les revendications 1 à 13.
